# EUROPEAN PATENT APPLICATION

(11) **EP 4 112 118 A1**
(43) Date of publication of application: **04.01.2023**
(21) Application number: 20930477.3
(22) Date of filing: 09.04.2020
(51) Int. Cl.: A61N 1/39

(54) **WCD SYSTEM AND MANAGEMENT METHOD THEREFOR**

(71) Applicant: Vivest Medical Technology Co., Ltd., Suzhou, Jiangsu 215000 (CN)
(72) Inventor: QIN, Zhihang, Suzhou, Jiangsu 215000 (CN); CHEN, Wusun, Suzhou, Jiangsu 215000 (CN); LIANG, Shengjin, Suzhou, Jiangsu 215000 (CN)
(74) Representative: De Arpe Tejero, Manuel
(86) International application number: PCT/CN2020/083951
(87) International publication number: WO 2021/203357

(57) **Abstract**

A WCD system, comprising: a collection module, a master control module, and a defibrillation module, where the collection module collects a signal and sends same to the master control module, and has human body motion detection and vibration prompting functions; the master control module has a VF/VT analysis algorithm to analyse the signal collected by the collection module, and can control a power supply of the defibrillation module; and the defibrillation module has the VF/VT analysis algorithm and a defibrillation control function. The collection module collects an electrocardiosignal and sends same to the master control module; the master control module performs analysis, and sends the signal to the defibrillation module when a defibrillation signal is analysed; the defibrillation module performs collection of the signal and performs VF/VT analysis; analysis results of the defibrillation module and the master control module are mutually used as a reference, a defibrillation discharging function having a high-reliability requirement is implemented in an independent defibrillation module, and an electrocardio collection function having a high real-time requirement is implemented in an independent collection module, so that the reliability of the system is improved.

## Description

### TECHNICAL FIELD

The present invention relates to the technical field of medical devices, in particular to a WCD system and a management method therefor.

### BACKGROUND

Sudden cardiac death (SCD) has become a very serious public health problem. Once it occurs, the survival rate of patients is very low. The survival rate in China is generally less than 1%. At present, the most effective preventive measure for SCD is the placement of an implantable cardioverter defibrillator (ICD), which is 99% effective. However, the placement of ICD has limitations in some aspects, for example, the high price, the need for surgery and the risk of infection. Some patients having the risk of SCD only in a specific period are not suitable for ICD placement. Some patients with high risk of SCD have contraindications to ICD placement.

A wearable cardioverter defibrillator (WCD) is a wearable external automated defibrillator. The patient wearing the WCD can be automatically given an electric shock therapy without bystander intervention, and the conscious patient can delay or terminate therapy by pressing a response button.

The WCD generally comprises the following modules: 1, wearable vest (comprising an electrocardio electrode, a defibrillation electrode and an electrocardio collection panel); 2, WCD main unit; 3, replaceable battery and charger.
1. Wearable vest: the vest is worn under the clothes and close to the skin, and an electrode belt surrounding the thorax and inside the vest is provided with four electrocardio electrodes which are in direct contact with the skin and form a collection system with two bipolar leads, which is used to record electrocardiosignals on the body surface and detect the patient's idioventricular rhythm and arrhythmia. The two sides of the midline of the back of the vest are each provided with one defibrillation panel, and the two defibrillation panels and one defibrillation electrode at the left precordium together form a defibrillation unit covering the surface of the heart. Each defibrillation electrode is provided with a "capsule" that automatically releases defibrillation gel. With the pressure of the chest strap and the waistband, the electrodes are kept in close contact with the chest wall. The electrocardio collection panel is mainly used to collect electrocardiosignals of the four electrocardio electrodes around the thorax and transmit in real time the electrocardiosignals to the WCD main unit for analysis and processing.
2. The WCD main unit is composed of a battery, a biphasic defibrillation wave module (a capacitor and a high-voltage transformer), a main control board, a wireless network module and a response button, and is carried on the body through shoulder straps. The WCD is in wired connection to the vest through a plug and a socket.
3. The replaceable battery is used to supply power to the WCD main unit, and the charger is used to charge the battery. One WCD main unit is equipped with at least 2 batteries with one being used for the WCD main unit, and the other one being charged on the charger. The battery is replaced regularly to ensure that the WCD main unit has sufficient electricity for defibrillation.

The WCD is worn by the patient outside the hospital for 3 days to a maximum of 6 months. The WCD main unit in a working state performs 24-hour electrocardio monitoring via the electrocardio electrodes. After detecting VF/VT (ventricular fibrillation/ventricular tachycardia) and confirming that the patient has no response, the WCD main unit switches to the defibrillation mode. The defibrillation electrode sprays conductive gel which is then in contact with the skin for defibrillation. The patients should be sent to the hospital as soon as possible after defibrillation, during which the electrocardio monitoring and the defibrillation function remain active.

In order to meet the needs of clinical application, the WCD main unit needs to perform functions such as electrocardio data collection, malignant arrhythmia analysis, wireless communication, voice prompts, keypress response and defibrillation discharging. If all the functions are implemented in one main control board, the design of embedded software will be very complicated, and the reliability of the system will be reduced. In order to solve this problem, a defibrillation discharging function having a high-reliability requirement is implemented in an independent defibrillation module, and an electrocardio collection function having a high real-time requirement is implemented in an independent collection module, so that the reliability of the system is improved.

The WCD is a wearable device. The patient wearing the WCD can move and even exercise. However, this may generate various complex interference signals, resulting in misjudgment of electrocardio analysis. In order to avoid mistaken defibrillation, the WCD main unit is provided with a response key. If VF/VT is detected, the main unit will broadcast a voice prompt of being ready for defibrillation, which reminds the patient to press the response key in time to cancel the defibrillation. If the patient does not develop VF/VT and is still conscious, he/she can press the response key in time to cancel the defibrillation. If the patient has developed VF/VT and has fallen into a coma, the WCD will initiate defibrillation after the end of the waiting time to treat the patient. If there is only one channel for electrocardio collection and analysis, there may be a high probability of misjudgment of VF/VT. In order to solve this problem, analysis results of a defibrillation module and a master control module are mutually used as a reference to determine the time for waiting the patient to press the response key to cancel the defibrillation, so that the accuracy of defibrillation response is increased.

### SUMMARY

### Technical problem

The technical problem to be solved by the present invention is: a defibrillation discharging function having a high-reliability requirement is implemented in an independent defibrillation module, and an electrocardio collection function having a high real-time requirement is implemented in an independent collection module, so that the reliability of the system is improved.

### Solutions to the problem

### Technical solutions

In order to solve the above technical problem, the present invention provides a WCD system, comprising: a collection module, a master control module and a defibrillation module.

The collection module collects a signal and sends the signal to the master control module, and has human body motion detection and vibration prompting functions.

The master control module has a VF/VT analysis algorithm to analyse the signal collected by the collection module, and is capable of controlling a power supply of the defibrillation module.

The defibrillation module has the VF/VT analysis algorithm and a defibrillation control function.

Further, the collection module comprises a collection MCU, the master control module comprises a master control MCU, the defibrillation module comprises a defibrillation MCU, and the master control MCU is in communication connection with the collection MCU and the defibrillation MCU.

Further, the collection MCU comprises a first data collection module, a data preprocessing module and a first data communication module.

The first data collection module is used for collecting electrocardio data of a patient and sending the electrocardio data to the data preprocessing module for further processing.

The data preprocessing module is used for performing preprocessing such as software filtering on the electrocardio data to enable the data to meet the analysis requirements of VF/VT, thereby obtaining preprocessed electrocardio data.

The first data communication module is used for sending the preprocessed electrocardio data to the master control MCU.

Further, the master control MCU comprises a second data communication module, a first electrocardio analysis module, a defibrillation processing module and an audio module.

The second data communication module is used for receiving and sending wireless network data.

The first electrocardio analysis module is used for analysing the electrocardio data sent from the collection MCU to judge whether VF/VT occurs.

The defibrillation processing module is used for processing state data sent from the defibrillation MCU.

The audio module is used for playing a voice prompt.

Further, the defibrillation MCU comprises a third data communication module, a gel release module, a second data collection module, a second electrocardio analysis module and a charging and discharging module.

The gel release module is used for controlling gel release of a defibrillation electrode.

The second data collection module is used for collecting electrocardio data from the defibrillation electrode.

The second electrocardio analysis module is used for analysing the electrocardio data sent from the second data collection module to judge whether VF/VT occurs.

The charging and discharging module is used for processing charging and discharging of a defibrillation capacitor.

The third data communication module is used for sending the state data of the defibrillation process to the master control MCU.

Further, the present invention further provides a management method for the WCD system according to any one of the above, comprising the following steps:
initialising, by the first data communication module, the second data communication module and the third data communication module, communication for connection;
collecting, by the collection MCU, electrocardio data and processing and analysing the electrocardio data;
receiving, by the master control MCU, the electrocardio data and performing the VF/VT detection; receiving and processing, by the master control MCU, state data of the defibrillation MCU and analysing the state data; and
performing, by the master control MCU, corresponding processing according to the different state data and shutting down the defibrillation MCU at the end of therapy.

Further, the collecting, by the collection MCU, the electrocardio data and processing and analysing the electrocardio data further comprises the following steps:
S110: collecting, by the first data collection module, the electrocardio data;
S120: performing, by the data preprocessing module, preprocessing such as software filtering on the electrocardio data to enable the electrocardio data to meet the analysis requirements of VF/VT; and
S130: sending, by the first data communication module, the preprocessed electrocardio data to the master control MCU.

Further, the receiving, by the master control MCU, the electrocardio data and performing the VF/VT detection; receiving and processing the state data of the defibrillation MCU and analysing the state data; and performing corresponding processing according to the different state data and shutting down the defibrillation MCU at the end of therapy further comprises the following steps:
S210: receiving, by the second data communication module, the electrocardio data from the collection MCU;
S220: performing, by the first electrocardio analysis module, the VF/VT detection on the electrocardio data, and starting the defibrillation MCU if occurrence of the VF/VT is detected;
S230: powering on, by the defibrillation processing module, the defibrillation module such that the defibrillation MCU starts to run;
S240: receiving, by the second data communication module, the state data of the defibrillation MCU and performing, by the second data communication module, corresponding processing on the received state data; and
S250: shutting down the defibrillation MCU at the end of the therapy.

Further, before receiving, by the second data communication module, the state data of the defibrillation MCU and performing, by the second data communication module, corresponding processing on the received state data, the method further comprises the following steps:
S310: detecting, by the gel release module, a connection state of the defibrillation electrode and sending the connection state to the master control MCU;
S320: releasing, by the gel release module, the gel and sending a gel release state to the master control MCU;
S330: collecting, by the second data collection module, the electrocardio data from the defibrillation electrode if the gel is released successfully;
S340: performing, by the second electrocardio analysis module, VF/VT analysis on the successfully collected electrocardio data and sending an analysis state to the master control MCU;
S350: if the analysis result is VF/VT electrocardio, charging, by the charging and discharging module, a charging capacitor and sending a charging state to the master control MCU; and
S360: if the charging is successful, discharging, by the charging and discharging module, the charging capacitor and sending a discharging state to the master control MCU.

Further, before powering on, by the defibrillation processing module, the defibrillation module such that the defibrillation MCU starts to run, the method further comprises:
playing, by the audio module, a starting failure prompt if the starting of the defibrillation MCU fails.

### Beneficial effects of the present invention

### Beneficial effects

The present invention has the following beneficial effects: the present invention provides a WCD system, comprising: a collection module, a master control module and a defibrillation module. The collection module collects a signal and sends the signal to the master control module, and has human body motion detection and vibration prompting functions. The master control module has a VF/VT analysis algorithm to analyse the signal collected by the collection module, and is capable of controlling a power supply of the defibrillation module. The defibrillation module has the VF/VT analysis algorithm and a defibrillation control function. The collection module collects an electrocardiosignal and sends the electrocardiosignal to the master control module; the master control module performs analysis, and sends the signal to the defibrillation module when a defibrillation signal is analysed; the defibrillation module performs collection of the signal and performs VF/VT analysis; analysis results of the defibrillation module and the master control module are mutually used as a reference, a defibrillation discharging function having a high-reliability requirement is implemented in an independent defibrillation module, and an electrocardio collection function having a high real-time requirement is implemented in an independent collection module, so that the reliability of the system is improved.

### BRIEF DESCRIPTION OF THE DRAWINGS

The specific structure of the present invention will be described in detail below in conjunction with the accompanying drawings.
FIG. 1 a block diagram of a WCD system according to the present invention;
FIG. 2 is a system block diagram of a collection module according to the present invention;
FIG. 3 is a system block diagram of a master control module according to the present invention;
FIG. 4 is a system block diagram of a defibrillation module according to the present invention;
FIG. 5 is a software block diagram according to the present invention;
FIG. 6 is a software flowchart of a master control MCU according to the present invention;
FIG. 7 is a software flowchart of a collection MCU according to the present invention; and
FIG. 8 is a software flowchart of a defibrillation MCU according to the present invention.

### DETAILED DESCRIPTION

In order to describe the technical contents, structural features, achieved objects and effects of the present invention in detail, the present invention will be described in detail below in conjunction with the embodiments and the accompanying drawings.

### Embodiment 1

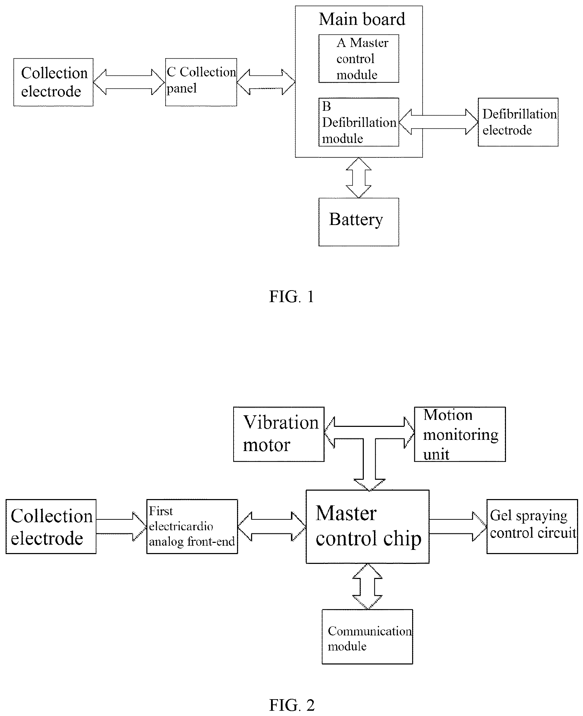

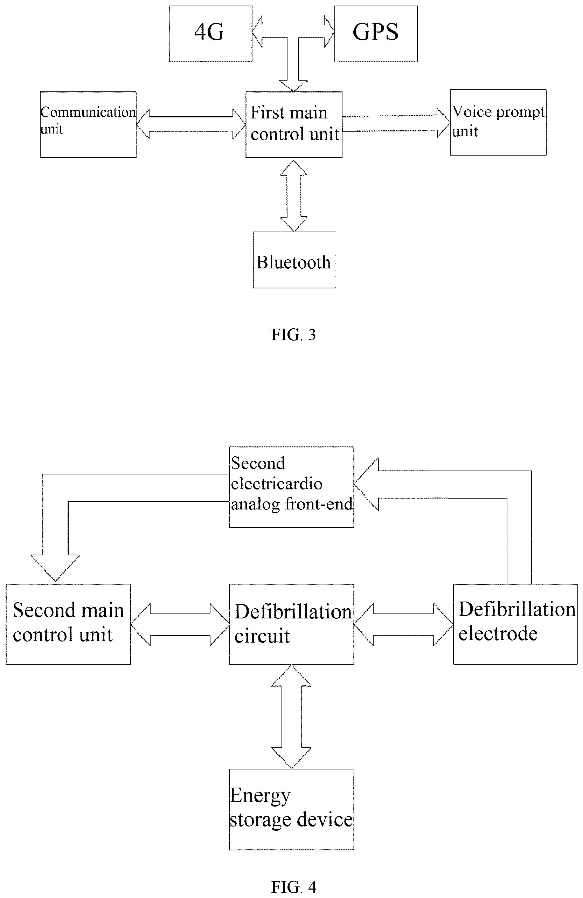

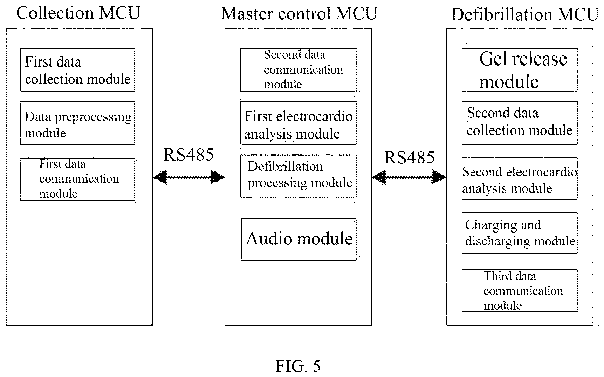

Referring to FIG. 1 to FIG. 5, the present invention provides a WCD system, comprising a collection module, a master control module and a defibrillation module. The collection module collects a signal and sends the signal to the master control module, and has human body motion detection and vibration prompting functions. The master control module has a VF/VT analysis algorithm to analyse the signal collected by the collection module, and is capable of controlling a power supply of the defibrillation module. The defibrillation module has the VF/VT analysis algorithm and a defibrillation control function. The collection module is an independent board, i.e., a collection panel. The master control module and the defibrillation module share one board, i.e., a main board. The collection module comprises a collection electrode, a first electricardio analog front-end, a master control chip, a vibration motor, a motion monitoring unit and a gel spraying control circuit. The master control chip is connected to the first electricardio analog front-end, the vibration motor, the motion monitoring unit and the gel spraying control circuit. The collection electrode is connected to the first electricardio analog front-end. The vibration motor and the motion monitoring unit are connected with each other. The collection electrode is a conventional conductive electrode and is used for signal collection. The first electricardio analog front-end adopts an integrated 6-channel electrocardio collection chip ADS1296, and has the advantages of simple external circuit and low power consumption. The master control chip adopts STM32L431CCUB, and is mainly used to collect signals of 4 electrodes in real time and send the signals to the main board for analysis, collect data of the motion monitoring unit for analysis at the same time, and control the vibration motor and the gel spraying control circuit. The vibration motor mainly adopts a driver IC that can drive LRA and ERM motors, and is used for vibration prompting. The motion monitoring unit adopts an acceleration sensor IC with a built-in machine learning kernel, and can effectively monitor the motion of the patient. The gel spraying control circuit is used for the conductive gel to be sprayed from the defibrillation electrode, so that the defibrillation electrode can be better in contact with the patient, thereby preventing the patient from being burned. The master control module comprises a communication unit, a first main control unit and a voice prompt unit. The first main control unit is connected to the communication unit and the voice prompt unit. The master control module is powered by a battery. The communication unit mainly adopts a 4G module, a GPS module and a Bluetooth module, and is used for positioning and real-time transmission of collected electricardio waveform data to a server. The chip of the first main control unit is STM32L4R5 with a built-in VF/VT algorithm, and is mainly used to analyse an electrocardiosignal collected by the collection panel. The voice prompt unit mainly uses a speaker for voice prompting. The defibrillation module comprises a defibrillation electrode, a defibrillation circuit, an energy storage device, a second electricardio analog front-end and a second main control unit. The second main control unit is connected to the second electricardio analog front-end and the defibrillation circuit. The defibrillation electrode is connected to the defibrillation circuit and the second electricardio analog front-end. The energy storage device is connected to the defibrillation circuit. The defibrillation electrode is a purpose-made defibrillation electrode. The defibrillation circuit comprises a charging and discharging circuit. The energy storage device is mainly a charging capacitor. The second electricardio analog front-end adopts a single-channel IC, and is used to collect the electrocardiosignal. The second main control unit uses STM32L431, which is mainly used to analyse the electrocardiosignal collected by the defibrillation electrode and control the defibrillation circuit.

The collection panel is powered by the main board, is capable of collecting the signals of the four electrodes at the same time and sending the signals to the main board through the RS485, and has human body motion detection and vibration prompting functions. The main board works continuously after a battery is inserted thereinto. The master control module has a VF/VT analysis algorithm, and is capable of analysing the signals collected by the collection panel. The master control module also has communication and voice prompting functions, and is capable of controlling a power supply of the defibrillation module. The defibrillation module has a defibrillation control function and the VF/VT analysis algorithm. After detecting the defibrillation signal, the master control module turns on the power supply of the defibrillation module and notifies the collection panel to make the gel spraying control circuit spray the conductive gel. Then the defibrillation module collects the electrocardiosignal through the defibrillation electrode making contact with the patient, and performs VF/VT analysis. Analysis results of the defibrillation module and the master control module may be mutually used as a reference to judge whether to perform defibrillation. If the defibrillation signal is confirmed, the defibrillation module activates the defibrillation circuit to complete defibrillation. A powered-off defibrillation module can consume less power. Besides, the defibrillation module is only responsible for defibrillation and has no other functions, which makes it more reliable.

Further, the collection module comprises a collection MCU, the master control module comprises a master control MCU, the defibrillation module comprises a defibrillation MCU, and the master control MCU is in communication connection with the collection MCU and the defibrillation MCU.

Further, the collection MCU comprises a first data collection module, a data preprocessing module and a first data communication module. The first data collection module is used for collecting electrocardio data of a patient and sending the electrocardio data to the data preprocessing module for further processing. The data preprocessing module is used for performing preprocessing such as software filtering on the electrocardio data to enable the data to meet the analysis requirements of VF/VT, thereby obtaining preprocessed electrocardio data. The first data communication module is mainly responsible for receiving and sending data of RS485, and mainly used for sending the preprocessed electrocardio data to the master control MCU.

Further, the master control MCU comprises a second data communication module, a first electrocardio analysis module, a defibrillation processing module and an audio module. The second data communication module comprises communication with the collection MCU and communication with the defibrillation MCU, and is mainly responsible for receiving and sending data of the RS485. The first electrocardio analysis module is used for analysing the electrocardio data sent from the collection MCU to judge whether VF/VT occurs. The defibrillation processing module is used for processing state data sent from the defibrillation MCU. The audio module is used for playing a voice prompt, comprising alarm voice, etc.

Further, the defibrillation MCU comprises a third data communication module, a gel release module, a second data collection module, a second electrocardio analysis module and a charging and discharging module. The gel release module is used for controlling gel release of the defibrillation electrode. The second data collection module is used for collecting electrocardio data from the defibrillation electrode. The second electrocardio analysis module is used for analysing the electrocardio data sent from the second data collection module to judge whether VF/VT occurs. The charging and discharging module is used for processing charging and discharging of a defibrillation capacitor. The third data communication module is mainly responsible for receiving and sending data of RS485, and mainly used for sending the state data of the defibrillation process to the master control MCU.

Further, the master control MCU comprises a keypress processing module. If the first electrocardio analysis module or the second electrocardio analysis module detects the occurrence of VF/VT, the audio module prompts the patient to press the response key to cancel the defibrillation. After waiting for a period of time Tl, for example 20 seconds, if the patient does not press the response key in time, then the power supply of the defibrillation module is turned on to defibrillate the patient. If the patient presses the response key in time, then the defibrillation is cancelled. If the first electrocardio analysis module and the second electrocardio analysis module detect the occurrence of VF/VT at the same time, the time for waiting the patient to press the response key is shortened, for example, to 10 seconds.

According to the WCD system provided by the present invention, a defibrillation discharging function having a high-reliability requirement is implemented in an independent defibrillation module, and an electrocardio collection function having a high real-time requirement is implemented in an independent collection module, so that the reliability of the system is improved. Analysis results of the defibrillation module and the master control module are mutually used as a reference to determine the time for waiting the patient to press the response key to cancel the defibrillation, so that the accuracy of defibrillation response is increased.

### Embodiment 2

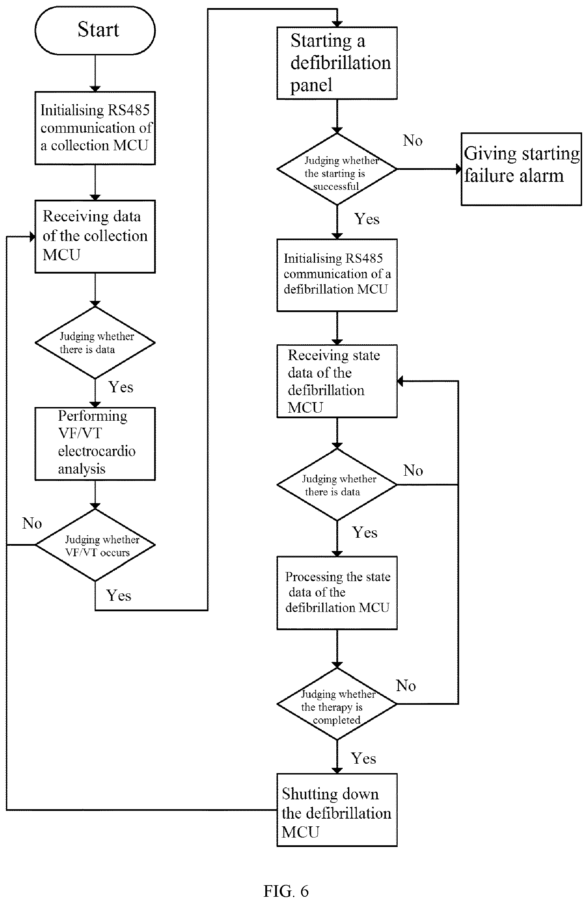

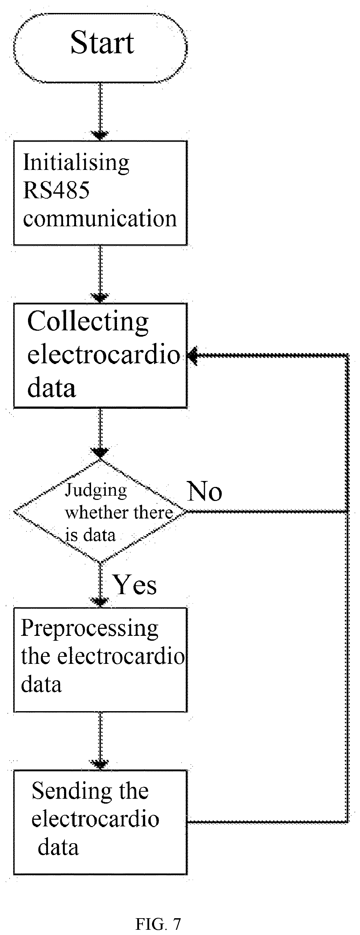

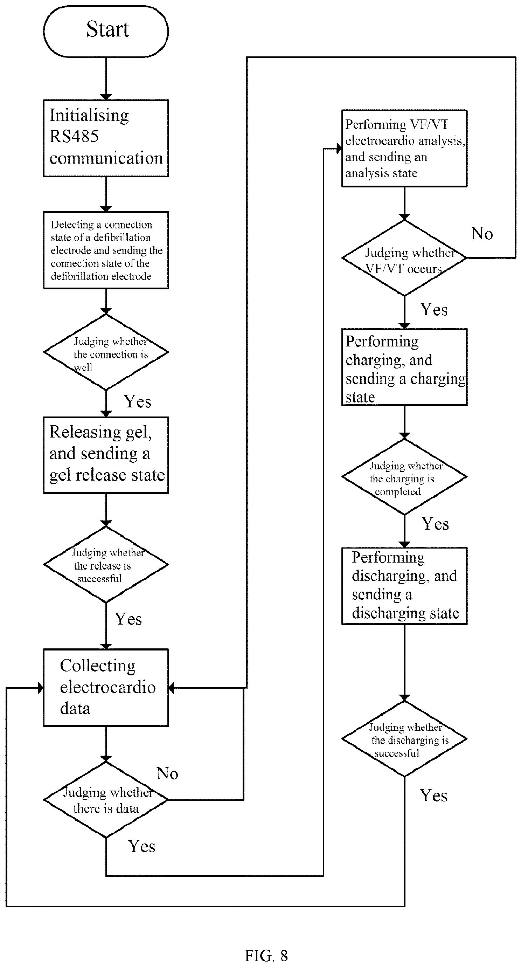

Referring to FIG. 6 to FIG. 8, the present invention further provides a management method for the WCD system of Embodiment 1, comprising the following steps:
initialising, by the first data communication module, the second data communication module and the third data communication module, communication for connection;
collecting, by the collection MCU, electrocardio data and processing and analysing the electrocardio data;
receiving, by the master control MCU, the electrocardio data and performing VF/VT detection; receiving and processing, by the master control MCU, state data of the defibrillation MCU and analysing the state data; and
performing, by the master control MCU, corresponding processing according to the different state data, activating a working process of the defibrillation MCU at the beginning of therapy, and deactivating the working process of the defibrillation MCU at the end of the therapy.

The first data communication module, the second data communication module and the third data communication module initialising communication for connection mainly comprises: the second data communication module initialises RS485 communication of the collection MCU for connection, and is used for receiving and sending data from the collection MCU; the first data communication module initialises RS485 communication of the master control MCU, and is mainly used for transmitting the electrocardio data to the master control MCU; the third data communication module initialises RS485 communication of the master control MCU, and is used for transmitting the state data to the master control MCU.

Further, collecting, by the collection MCU, the electrocardio data and processing and analysing the electrocardio data further comprises the following steps:
S110: collecting, by the first data collection module, the electrocardio data from the ADS 1296 chip;
S120: performing, by the data preprocessing module, preprocessing such as software filtering on the electrocardio data to enable the electrocardio data to meet the analysis requirements of VF/VT; and
S130: sending, by the first data communication module, the preprocessed electrocardio data to the master control MCU.

Further, receiving, by the master control MCU, the electrocardio data and performing VF/VT detection; receiving and processing state data of the defibrillation MCU and analysing the state data; performing corresponding processing according to the different state data, turning on a power supply of the defibrillation MCU at the beginning of therapy, and shutting down the defibrillation MCU at the end of the therapy further comprises the following steps:
S210: receiving, by the second data communication module, the electrocardio data from the collection MCU;
S220: performing, by the first electrocardio analysis module, the VF/VT detection on the electrocardio data from the collection MCU and starting the defibrillation MCU and activating the working process of the defibrillation MCU if occurrence of the VF/VT is detected, otherwise, proceeding to S210;
S230: powering on, by the defibrillation processing module, the defibrillation module on the main board to activate the defibrillation function such that the defibrillation MCU starts to run;
S240: initialising, by the second data communication module, RS485 communication of the defibrillation MCU for connection, and receiving the state data of the defibrillation MCU, comprising a gel release state, an electrocardio analysis state, a charging or discharging state, etc., and performing, by the defibrillation processing module, corresponding processing on the received state data, wherein for example, if the gel is being released, then the audio module plays a voice of being in the gel release state; if the electrocardio analysis is in progress, then the audio module plays a voice of being in the electrocardio analysis state; and if charging is being performed, then the audio module plays a voice of being in the charging state; and
S250: shutting down the defibrillation MCU if the end of the therapy is detected, wherein after the defibrillation processing module turns off the power supply of the defibrillation module on the main board, and the defibrillation MCU stops running.

Further, before receiving, by the second data communication module, the state data of the defibrillation MCU and performing, by the defibrillation processing module, the corresponding processing on the received state data, the method further comprises the following steps:
S310: detecting, by the gel release module, a connection state of the defibrillation electrode and sending the connection state to the master control MCU through the third data communication module;
S320: releasing, by the gel release module, the gel and sending a gel release state to the master control MCU through the third data communication module if it is detected that the connection of the defibrillation electrode is well;
S330: collecting, by the second data collection module, the electrocardio data from the defibrillation electrode if the gel is released successfully;
S340: performing, by the second electrocardio analysis module, VF/VT analysis on the successfully collected electrocardio data and sending an analysis state to the master control MCU through the third data communication module;
S350: if the analysis result is VF/VT electrocardio, charging, by the charging and discharging module, a charging capacitor and sending a charging state to the master control MCU, otherwise proceeding to S330; and
S360: if the charging is successful, discharging, by the charging and discharging module, the charging capacitor and sending a discharging state to the master control MCU, and proceeding to S330 after the discharging is successful.

Further, before powering on, by the defibrillation processing module, the defibrillation module such that the defibrillation MCU starts to run, the method further comprises:
playing, by the audio module, a starting failure prompt if the starting of the defibrillation MCU fails.

Further, the master control MCU comprises a keypress processing module. If the first electrocardio analysis module or the second electrocardio analysis module detects the occurrence of VF/VT, the audio module prompts the patient to press the response key to cancel the defibrillation. After waiting for a period of time T1, for example 20 seconds, if the patient does not press the response key in time, the power supply of the defibrillation module is turned on to defibrillate the patient. If the patient presses the response key in time, then the defibrillation is cancelled. If the first electrocardio analysis module and the second electrocardio analysis module detect the occurrence of VF/VT at the same time, the time for waiting the patient to press the response key is shortened, for example, to 10 seconds.

Based on the above, according to the WCD system provided by the present invention, a defibrillation discharging function having a high-reliability requirement is implemented in an independent defibrillation module, and an electrocardio collection function having a high real-time requirement is implemented in an independent collection module, so that the reliability of the system is improved. Analysis results of the defibrillation module and the master control module are mutually used as a reference to determine the time for waiting the patient to press the response key to cancel the defibrillation, so that the accuracy of defibrillation response is increased.

The terms "first", "second", ..., herein represent only the distinction of their names, not the difference in their importance and position.

The terms "upper", "lower", "left", "right", "front" and "rear" represent only their relative positions but not their absolute positions.

The above only describes the embodiments of the present invention, and is not intended to limit the scope of the present invention. Any equivalent structure or equivalent process transformation made by using the contents of the description and accompanying drawings of the present invention or directly or indirectly applied in other related technical fields are similarly included in the scope of patent protection of the present invention.

## Claims

1. A WCD system, **characterized by** comprising a collection module, a master control module and a defibrillation module; wherein
the collection module collects a signal and sends the signal to the master control module, and has human body motion detection and vibration prompting functions;
the master control module has a VF/VT analysis algorithm to analyse the signal collected by the collection module, and is capable of controlling a power supply of the defibrillation module; and
the defibrillation module has the VF/VT analysis algorithm and a defibrillation control function.

2. The WCD system according to claim 1, **characterized in that** the collection module comprises a collection MCU, the master control module comprises a master control MCU, the defibrillation module comprises a defibrillation MCU, and the master control MCU is in communication connection with the collection MCU and the defibrillation MCU.

3. The WCD system according to claim 2, **characterized in that** the collection MCU comprises a first data collection module, a data preprocessing module and a first data communication module, wherein
the first data collection module is used for collecting electrocardio data of a patient and sending the electrocardio data to the data preprocessing module for further processing;
the data preprocessing module is used for performing preprocessing such as software filtering on the electrocardio data to enable the data to meet the analysis requirements of VF/VT, thereby obtaining preprocessed electrocardio data; and
the first data communication module is used for sending the preprocessed electrocardio data to the master control MCU.

4. The WCD system according to claim 3, **characterized in that** the master control MCU comprises a second data communication module, a first electrocardio analysis module, a defibrillation processing module and an audio module, wherein
the second data communication module is used for receiving and sending wireless network data;
the first electrocardio analysis module is used for analysing the electrocardio data sent from the collection MCU to judge whether VF/VT occurs;
the defibrillation processing module is used for processing state data sent from the defibrillation MCU; and
the audio module is used for playing a voice prompt.

5. The WCD system according to claim 4, **characterized in that** the defibrillation MCU comprises a third data communication module, a gel release module, a second data collection module, a second electrocardio analysis module and a charging and discharging module, wherein
the gel release module is used for controlling gel release of a defibrillation electrode;
the second data collection module is used for collecting electrocardio data from the defibrillation electrode;
the second electrocardio analysis module is used for analysing the electrocardio data sent from the second data collection module to judge whether VF/VT occurs;
the charging and discharging module is used for processing charging and discharging of a defibrillation capacitor; and
the third data communication module is used for sending the state data of the defibrillation process to the master control MCU.

6. A management method for the WCD system according to any one of claims 1 to 5, **characterized by** comprising the following steps:
initialising, by the first data communication module, the second data communication module and the third data communication module, communication for connection;
collecting, by the collection MCU, electrocardio data and processing and analysing the electrocardio data;
receiving, by the master control MCU, the electrocardio data and performing VF/VT detection; receiving and processing, by the master control MCU, state data of the defibrillation MCU and analysing the state data; and
performing, by the master control MCU, corresponding processing according to the different state data and shutting down the defibrillation MCU at the end of therapy.

7. The management method for the WCD system according to claim 6, **characterized in that** the collecting, by the collection MCU, the electrocardio data and processing and analysing the electrocardio data further comprises the following steps:
S110: collecting, by the first data collection module, the electrocardio data;
S120: performing, by the data preprocessing module, preprocessing such as software filtering on the electrocardio data to enable the electrocardio data to meet the analysis requirements of VF/VT; and
S130: sending, by the first data communication module, the preprocessed electrocardio data to the master control MCU.

8. The management method for the WCD system according to claim 6, **characterized in that** the receiving, by the master control MCU, the electrocardio data and performing the VF/VT detection; receiving and processing the state data of the defibrillation MCU and analysing the state data; and performing corresponding processing according to the different state data and shutting down the defibrillation MCU at the end of therapy further comprises the following steps:
S210: receiving, by the second data communication module, the electrocardio data from the collection MCU;
S220: performing, by the first electrocardio analysis module, the VF/VT detection on the electrocardio data, and starting the defibrillation MCU if occurrence of the VF/VT is detected;
S230: powering on, by the defibrillation processing module, the defibrillation module such that the defibrillation MCU starts to run;
S240: receiving, by the second data communication module, the state data of the defibrillation MCU and performing, by the defibrillation processing module, corresponding processing on the received state data; and
S250: shutting down the defibrillation MCU at the end of the therapy.

9. The management method for the WCD system according to claim 8, **characterized in that** before receiving, by the second data communication module, the state data of the defibrillation MCU and performing, by the defibrillation processing module, corresponding processing on the received state data, the method further comprises the following steps:
S310: detecting, by the gel release module, a connection state of the defibrillation electrode and sending the connection state to the master control MCU;
S320: releasing, by the gel release module, the gel and sending a gel release state to the master control MCU;
S330: collecting, by the second data collection module, the electrocardio data from the defibrillation electrode if the gel is released successfully;
S340: performing, by the second electrocardio analysis module, VF/VT analysis on the successfully collected electrocardio data and sending an analysis state to the master control MCU;
S350: if the analysis result is VF/VT electrocardio, charging, by the charging and discharging module, the charging capacitor and sending a charging state to the master control MCU; and
S360: if the charging is successful, discharging, by the charging and discharging module, the charging capacitor and sending a discharging state to the master control MCU.

10. The management method for the WCD system according to claim 8, **characterized in that** before powering on, by the defibrillation processing module, the defibrillation module such that the defibrillation MCU starts to run, the method further comprises:
playing, by the audio module, a starting failure prompt if the starting of the defibrillation MCU fails.
